(19) Europäisches Patentamt
European Patent Office
Office européen des brevets

(11) Veröffentlichungsnummer : **0 502 013 B1**

(12) # EUROPÄISCHE PATENTSCHRIFT

(45) Veröffentlichungstag der Patentschrift :
**20.04.94 Patentblatt 94/16**

(51) Int. Cl.$^5$ : **C11D 3/39,** C11D 3/42

(21) Anmeldenummer : **90917030.0**

(22) Anmeldetag : **13.11.90**

(86) Internationale Anmeldenummer :
**PCT/EP90/01897**

(87) Internationale Veröffentlichungsnummer :
**WO 91/08279 13.06.91 Gazette 91/13**

(54) **HETEROCYCLISCHE VERBINDUNGEN ALS BLEICHAKTIVATOREN ODER OPTISCHE AUFHELLER IN WASCHMITTELN.**

(30) Priorität : **21.11.89 DE 3938526**

(43) Veröffentlichungstag der Anmeldung :
**09.09.92 Patentblatt 92/37**

(45) Bekanntmachung des Hinweises auf die Patenterteilung :
**20.04.94 Patentblatt 94/16**

(84) Benannte Vertragsstaaten :
**AT BE CH DE DK ES FR GB GR IT LI LU NL SE**

(56) Entgegenhaltungen :
**EP-A- 0 314 350**
**EP-A- 0 332 294**
**FR-A- 1 392 448**
**FR-A- 1 513 274**

(73) Patentinhaber : **BASF Aktiengesellschaft**
**Carl-Bosch-Strasse 38**
**D-67063 Ludwigshafen (DE)**

(72) Erfinder : **DUNG, Bernhard**
**Koenigsberger Strasse 4**
**D-6718 Gruenstadt (DE)**
Erfinder : **TRIESELT, Wolfgang**
**Alwin-Mittasch-Platz 1**
**D-6700 Ludwigshafen (DE)**
Erfinder : **HAHN, Erwin**
**Am Buechsenackerhang 31**
**D-6900 Heidelberg (DE)**
Erfinder : **PERNER, Johannes**
**Ginsterweg 4**
**D-6730 Neustadt (DE)**
Erfinder : **OFTRING, Alfred**
**Im Roehrich 49**
**D-6702 Bad Duerkheim (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 502 013 B1

Jouve, 18, rue Saint-Denis, 75001 PARIS

## Beschreibung

Die vorliegende Erfindung betrifft die Verwendung von heterocyclischen Verbindungen der allgemeinen Formel I

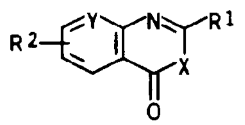

I

in der die Variablen folgende Bedeutung haben

X  0, S oder die Gruppierung N-$R^3$, wobei $R^3$ für Wasserstoff, $C_1$-$C_{25}$-Alkyl; $C_1$-$C_{25}$-Acyl oder eine Arylgruppe mit bis zu 12 C-Atomen steht,

Y  CH oder N,

$R^1$  für X = S oder N-$R^3$
Wasserstoff, eine $C_1$-$C_{25}$-Alkyl- oder $C_2$-$C_{25}$-Alkengruppe oder ein Phenylrest, der zusätzlich durch eine oder zwei $C_1$-$C_4$-Alkyl-, $C_1$-$C_4$-Alkoxy-, Hydroxyl-, Carboxyl-, Sulfo-, Amino-, $C_1$-$C_4$-Acylamino-, Nitro- oder Cyanogruppen oder Chlor- oder Bromatome substituiert sein kann, wobei bei zwei Substituenten diese gleich oder verschieden sein können,

$R^1$  für X = 0
Phenyl, o-, m- oder p-Tolyl, p-Chlorphenyl, m-Nitrophenyl, m-Methoxyphenyl oder m-Methylsulfonylphenyl,

$R^2$  für X = s oder N-$R^3$
Wasserstoff, eine $C_1$-$C_4$-Alkyl-, $C_1$-$C_4$-Alkoxy-, Hydroxyl-, Carboxyl-, Sulfo-, Amino- oder $C_1$-$C_4$-Acylaminogruppe oder ein Chlor- oder Bromatom und

$R^2$  für X = 0
Wasserstoff

als Bleichaktivatoren oder optische Aufheller in Wasch- und Reinigungsmitteln.

Außerdem betrifft die Erfindung Wasch- und Reinigungsmittel, die die Verbindungen I enthalten.

In der EP-B 099 197 (1) und der EP-B 240 057 (2) werden als übliche Bleichaktivatoren für Wasch- und Reinigungsmittel unter anderem acylierte Amine wie Tetraacetylethylendiamin (TAED), acylierte Zucker wie Pentaacetylglucose, Carbonsäureester wie Natrium-p-acetoxybenzolsulfonat sowie eine Reihe acylierter heterocyclischer Verbindungen, nämlich Hydantoine, cyclische Hydrazide, Triazole, Urazole, Imidazoline, Glycolurile, Piperazine und cyclische Harnstoffe genannt.

In der US-A 3 822 114 (3) werden als Bleichaktivatoren Aldehyd- und Ketonverbindungen beschrieben. Als Beispiele für heterocyclische Ketone werden dort Piperidin-Derivate sowie Tetrahydrothiopyranon- und 4-Oxacyclohexanon-Derivate genannt.

In der Literaturstelle J. Org. Chem. Bd. 14, S. 967-981 (1949) (4) wird die Herstellung von Benz-(4H)1,3-oxazin-4-on und in der 2-Stellung substituierten Derivaten beschrieben. Als Substituenten werden Methyl, Ethyl, n-Propyl, Phenyl, o- und p-Tolyl, o- und p-Chlorphenyl, o- und p-Nitrophenyl sowie 3-Pyridyl genannt. über Anwendungseigenschaften dieser Verbindungen wird keine Aussage gemacht.

Die EP-A 332 294 (5) betrifft Waschmittelzusammensetzungen, die u.a. Benz-(4H)1,3-oxazin-4-one der Formel I (X = 0, Y= CH, $R^2$ = H) als Bleichaktivatoren enthalten, wobei $R^1$ für Wasserstoff, Alkyl, Alkaryl, Aryl, Aralkyl, Alkoxyl, Halogenalkyl, Amino, Aminoalkyl, Carboxyl oder eine carboxylhaltige Gruppe steht. In der Beschreibung und den Beispielen werden ausschließlich nur solche Verbindungen genannt, bei denen $R^1$ Alkyl, insbesondere Methyl, Amino, Aminoalkyl, Acyl, Alkoxyl, Halogenalkyl, Alkoxyalkylenethergruppen oder Alkylencarboxylatgruppen bezeichnen.

Die Bleichaktivatoren des Standes der Technik haben sich als noch verbesserungsbedürftig erwiesen. Insbesondere ist oftmals die Einsatzmenge dieser Mittel, die notwendig ist, um eine ausreichende Wirksamkeit zu erzielen, in den Wasch- und Reinigungsmittel zu hoch.

Aufgabe der vorliegenden Erfindung war es deshalb, Bleichaktivatoren bereitzustellen, die bei geringerer Einsatzmenge die gleiche Wirkung wie die Mittel des Standes der Technik entfalten.

Demgemäß wurde die Verwendung der eingangs genannten heterocyclischen Verbindungen I für diesen Zweck gefunden.

Die erfindungsgemäß zu verwendenden Verbindungen I gehören für den Fall Y = CH zu den Substanzklassen der Benz-(4H)1,3-oxazin-4-one (X = O), Benz-(4H)1,3-thiazin-4-one (X = S) und (4H)1,3-Chinazolin-4-one (X = N-$R^3$) oder zu den entsprechenden pyridoanellierten Verbindungen (Y = N).

In einer bevorzugten Ausführungsform werden Benz-(4H)1,3-oxazin-4-on-Derivate (Y = CH) eingesetzt, bei denen $R^1$ die für den Fall X = O angegebene Bedeutung hat. Besonders bevorzugt wird die Verwendung von 2-(p-Tolyl)-, 2-(p-Chlorphenyl)-, 2-(m-Nitrophenyl)-, 2-(p-Methylsulfonylphenyl)- und vor allem 2-Phenyl-benz-(4H)1,3-oxazin-4-on.

In einer weiteren bevorzugten Ausführungsform werden Benz-(4H)1,3-thiazin-4-on (Y = CH) und (4H)1,3-Chinazolin-4-on (Y = CH) sowie ihre Derivate, bei denen $R^1$ und $R^2$ die für den Fall X = S oder N-$R^3$ angegebenen Bedeutungen haben, eingesetzt.

Als Reste $R^3$ kommen $C_1$-$C_{25}$-Alkylgruppen, vorzugsweise $C_1$-$C_{10}$-Alkylgruppen, z.B. Methyl, Ethyl, n-Propyl, n-Butyl, 2-Ethylhexyl oder Isononyl, $C_1$-$C_{25}$-Acylgruppen, vorzugsweise $C_1$-$C_{10}$-Acylgruppen, beispielsweise Formyl, Acetyl, Propionyl, Butyryl oder Octanoyl, oder eine Arylgruppe mit bis zu 12 C-Atomen wie Naphthyl, Biphenyl, Tolyl, Xylyl oder insbesondere Phenyl in Betracht.

Als Reste $R^1$ sind für den Fall X = S oder N-$R^3$ neben Wasserstoff zu nennen:

- $C_1$-$C_{25}$-Alkylgruppen, vorzugsweise $C_1$-$C_{10}$-Alkylgruppen, welche geradkettig oder verzweigt sein können, beispielsweise Methyl, Ethyl, n-Propyl, n-Butyl, tert.-Butyl, n-Pentyl, n-Hexyl, n-Heptyl, 1-Ethylpentyl, n-Octyl, 2,4,4-Trimethylpentyl, n-Nonyl, n-Decyl, n-Undecyl, n-Tridecyl, n-Pentadecyl und n-Heptadecyl
- $C_2$-$C_{25}$-Alkenylgruppen, vorzugsweise $C_2$-$C_6$- und $C_{15}$-$C_{21}$-Alkenylgruppen, beispielsweise Vinyl, 1-Propenyl, 2-Propenyl, Heptadec-8-enyl, Heptadeca-8,11-dienyl und Heptadeca-8,11,14-trienyl
- ein Phenylrest, der zusätzlich einen oder zwei, vorzugsweise einen Substituenten tragen kann, beispielsweise Phenyl, o-, m- oder p-Tolyl, 2,3-, 2,4-, 2,5-, 2,6-, 3,4- oder 3,5 Dimethylphenyl, o-, m- oder p-Ethylphenyl, m- oder p-tert.-Butylphenyl, o-, m- oder p-Methoxyphenyl, 2,3-, 2,4-, 2,5-, 2,6-, 3,4- oder 3,5-Dimethoxyphenyl, o-, m-oder p-Hydroxyphenyl, 3-Hydroxy-4-methoxyphenyl, o-, m- oder p-Carboxyphenyl, o-, m-oder p-Sulfophenyl, o-, m- oder p-Aminophenyl, o-, m- oder p-Acetaminophenyl, o-, m- oder p-Nitrophenyl, o-, m- oder p-Cyanophenyl, o-, m- oder p-Chlorphenyl und o-, m- oder p-Bromphenyl.

Als Reste $R^2$ kommen für den Fall X = S oder N-$R^3$ neben Wasserstoff eine $C_1$-$C_4$-Alkylgruppe, z.B. Methyl, Ethyl, n-Propyl, iso-Propyl, n-Butyl oder tert.-Butyl, eine $C_1$-$C_4$-Alkoxygruppe, z.B. Methoxy, Ethoxy, n-Butoxy oder tert.-Butoxy, eine Hydroxyl-, Carboxyl-, Sulfo-, Amino- oder $C_1$-$C_4$-Acylaminogruppe, z.B. Acetamino, Propionylamino oder Butyrylamino, sowie ein Chlor- oder Bromatom in Betracht. Diese Substituenten können in der 5-, 6-, 7- oder 8-Positionen stehen.

Bevorzugte Reste $R^2$ sind hierbei Wasserstoff, eine $C_1$-$C_4$-Alkyl-, $C_1$-$C_4$-Alkoxy- oder eine Hydroxylgruppe.

Die Verbindungen I eignen sich in hervorragender Weise als Bleichaktivatoren oder als optische Aufheller für Wasch- und Reinigungsmittel, insbesondere bei der Wäsche von Haushaltstextilien bei Temperaturen von 30 bis 60°C. Man benötigt zumeist nur noch ein Drittel der Menge an I als Bleichaktivator, verglichen mit den üblichen Bleichaktivatoren, um mit den Verbindungen I die gleiche Wirkung wie mit diesen zu erzielen.

Weiterhin sind Gegenstand der vorliegenden Erfindung Wasch- und Reinigungsmittel, die 0,1 bis 10 Gew.-%, vorzugsweise 0,5 bis 6 Gew.-%, bezogen auf die Gesamtmenge der Zubereitung, einer oder mehrerer heterocyclischer Verbindungen I enthalten.

Zur Verbesserung der Bleichaktivatorwirkung können die Wasch- und Reinigungsmittel noch weitere übliche Bleichaktivatoren in den hierfür üblichen Mengen enthalten.

Als zusätzliche Bleichaktivatoren kommen vor allem in Betracht:

- polyacylierte Zucker, z.B. Pentaacetylglucose;
- Acyloxybenzolsulfonsäuren und deren Alkali- und Erdalkalimetallsalze, z.B. Natrium-p-isononanoyloxy-benzolsulfonat oder Natrium-p-benzoyloxy-benzolsulfonat;
- N-diacylierte und N,N'-tetraacylierte Amine, z.B. N,N,N',N'-Tetraacetyl-methylendiamin und -ethylendiamin, N,N-Diacetylanilin, N,N-Diacetyl-p-toluidin oder 1,3-diacylierte Hydantoine wie 1,3-Diacetyl-5,5-dimethylhydantoin;

Daneben kommen aber auch noch als zusätzliche Bleichaktivatoren in Frage:

- N-Alkyl-N-sulfonyl-carbonamide, z.B. N-Methyl-N-mesyl-acetamid oder N-Methyl-N-mesyl-benzamid;
- N-acylierte cyclische Hydrazide, acylierte Triazole oder Ürazole, z.B. Monoacetyl-maleinsäurehydrazid;
- O,N,N-trisubstituierte Hydroxylamine, z.B. O-Benzoyl-N,N-succinylhydroxylamin, O-Acetyl-N,N-succinyl-hydroxylamin oder O,N,N-Triacetylhydroxylamin;
- N,N'-Diacyl-sulfurylamide, z.B. N,N'-Dimethyl-N,N'-diacetyl-sulfurylamid oder N,N'-Diethyl-N,N'-dipropionyl-sulfurylamid;
- Triacylcyanurate, z.B. Triacetylcyanurat oder Tribenzoylcyanurat;
- Carbonsäureanhydride, z.B. Benzoesäureanhydrid, m-Chlorbenzoesäureanhydrid oder Phthalsäureanhydrid;
- 1,3-Diacyl-4,5-diacyloxy-imidazoline, z.B. 1,3-Diacetyl-4,5-diacetoxyimidazolin;

- Tetraacetylglycoluril und Tetrapropionylglycoluril;
- diacylierte 2,5-Diketopiperazine, z.B. 1,4-Diacetyl-2,5-diketopiperazin
- Acylierungsprodukte von Propylendiharnstoff und 2,2-Dimethylpropylendiharnstoff, z.B. Tetraacetylpropylendiharnstoff;
- α-Acyloxy-polyacyl-malonamide, z.B. α-Acetoxy-N,N'-diacetylmalonamid;
- Diacyl-dioxohexahydro-1,3,5-triazine, z.B. 1,5-Diacetyl-2,4-dioxohexahydro-1,3,5-triazin.

Wasch- und Reinigungsmittelformulierungen mit den Verbindungen I und gegebenenfalls weiteren üblichen Bleichaktivatoren enthalten in der Regel als zusätzliche Bestandteile, bezogen auf das Gesamtgewicht der Formulierung, 6 bis 25 Gew.-% Tenside, 15 bis 50 Gew.-% Builder und gegebenenfalls Co-Builder, 10 bis 30 Gew.-% Bleichmittel und 5 bis 30 Gew.-% Hilfsstoffe wie Enzyme, Schaumregulatoren, Korrosionsinhibitoren, weitere optische Aufheller, Duftstoffe, Farbstoffe oder Formulierungshilfsmittel wie z.B. Natriumsulfat in den hierfür üblichen Mengen. Die genaue Spezifikation dieser Bestandteile ist dem Fachmann bekannt und braucht deshalb hier nicht näher aufgeführt zu werden.

Beispiele

Anwendung von Benz-(4H)1,3-oxazin-4-onen I als Bleichaktivatoren in Haushaltswaschmitteln

Waschmittelzubereitungen der Zusammensetzung

| | |
|---|---|
| 6,25 Gew.-% | Natriumdodecylbenzolsulfonat |
| 4,7 Gew.-% | ethoxylierter $C_{13}$-$C_{15}$-Oxoalkohol mit 7 mol Ethylenoxid |
| 2,8 Gew.-% | Talgfettseife |
| 1,0 Gew.-% | Carboxymethylcellulose |
| 2,0 Gew.-% | Copolymer aus Maleinsäure und Acrylsäure (Gewichtsverhältnis 30 : 70, mittlere Molmasse 70.000) |
| 4,5 | Gew.-% Natriumdisilicat |
| 1,0 | Gew.-% Magnesiumsilicat |
| 25,0 | Gew.-% Zeolith A |
| 10,0 | Gew.-% Natriumperborat-Monohydrat |
| x Gew.-% | Bleichaktivator |
| (30,75-x) Gew.-% | Natriumsulfat |

wurden auf ihre Eignung für die Textilwäsche untersucht.

Die Menge x und die Art des Bleichaktivators sind in der Tabelle angegeben.

Von diesem Testwaschmittel wurden jeweils 8 g/l Waschflotte eingesetzt.

Folgende Waschbedingungen wurden angewendet:

Als Waschgerät wurde das Launder-O-meter der Firma Atlas verwendet.

Die Wasserhärte des Waschwassers betrug 16,8°d ≙ 3 mmol $Ca^{2+}$/1 ($ca^{2+}$ : $Mg^{2+}$ = 3 : 1).

Die Waschzeit einschlieplich Aufheizzeit betrug jeweils 30 Minuten und das Flottenverhältnis 1 : 12,5.

Es wurden folgende Testgewebe verwendet:

A: Bauwoll-Gewebe mit Immedialschwarz als Test-Anschmutzung (Testgewebe EMPA 115)

B: Polyester-Gewebe mit Carotin/Speiseöl-Anschmutzung

Ermittelt wurde jeweils der Bleichgrad aufgrund photometrischer Messung der Remissiongrade der Gewebe vor und nach der Wäsche im Elrepho (Datacolor) der Firma Zeiss bei einer Wellenlänge von 460 nm (Bariumweißstandard nach DIN 5033).

Für die Berechnung des Bleichgrades BG gilt folgende Beziehung:

$$BG = \frac{F_v - F_n}{F_v - F_o} \times 100$$

wobei für die Farbstärke F (= Schmutzigkeit des Gewebes) die Kubelka-Munk-Beziehung gilt:

$$F = \frac{(1-R)^2}{2R}$$

dabei bedeuten

R: Remissionsgrad (Meßgröße)
v: vor dem Waschen des angeschmutzten Testgewebes
n: nach dem Waschen des angeschmutzten Testgewebes
o: vor dem Anschmutzen des Gewebes.

Die Tabelle zeigt die errechneten Bleichgrade der Testgewebe jeweils nach einer einmaligen Wäsche bei

20°C, 38°C oder 60°C.

Tabelle

Bleichgrade für Testgewebe A und B

| Bleichaktivator | Menge x [Gew.-%] | Bleichgrad BG bei Testgewebe A 20°C | 38°C | 60°C | Testgewebe B 20°C | 38°C | 60°C |
|---|---|---|---|---|---|---|---|
| erfindungsgemäß: | | | | | | | |
| 2-Phenyl-benz-(4H)1,3-oxazin-4-on | 6 | 24 | 42 | 58 | 61 | 78 | 77 |
| 2-(o-Tolyl)benz-(4H)1,3-oxazin-4-on | 6 | 19 | 32 | 48 | 63 | 77 | 79 |
| 2-(m-Tolyl)benz-(4H)1,3-oxazin-4-on | 6 | 19 | 29 | 43 | 62 | 76 | 77 |
| 2-(p-Tolyl)benz-(4H)1,3-oxazin-4-on | 6 | 25 | 44 | 62 | 65 | 78 | 78 |
| 2-(p-Chlorphenyl)benz-(4H)1,3-oxazin-4-on | 6 | 31 | 48 | 60 | 73 | 83 | 86 |
| 2-(m-Nitrophenyl)benz-(4H)1,3-oxazin-4-on | 6 | 23 | 42 | 63 | 61 | 76 | 77 |
| 2-(m-Methoxyphenyl)benz-(4H)1,3-oxazin-4-on | 6 | 22 | 34 | 45 | 60 | 72 | 74 |
| 2-(m-Methylsulfonylphenyl)benz-(4H)1,3-oxazin-4-on | 6 | 42 | 53 | 57 | 59 | 72 | 76 |
| zum Vergleich: | | | | | | | |
| 2-Methyl-benz-(4H)1,3-oxazin-4-on* | 6 | 15 | 25 | 43 | 58 | 70 | 73 |
| Tetraacetylethylendiamin (TAED) | 6 | 14 | 20 | 24 | 60 | 65 | 69 |
| ohne Bleichaktivator | 0 | 8 | 13 | 19 | 61 | 62 | 69 |

* gemäß EP-A 332 294 (5)

## Patentansprüche

## Patentansprüche für folgende Vertragsstaaten : AT, BE, CH, DE, DK, FR, GB, GR, IT, LI, LU, NL, SE

1. Verwendung von heterocyclischen Verbindungen der allgemeinen Formel I

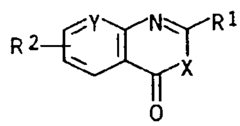

I

in der die Variablen folgende Bedeutung haben

X O, S oder die Gruppierung N-$R^3$, wobei $R^3$ für Wasserstoff, $C_1$-$C_{25}$-Alkyl, $C_1$-$C_{25}$-Acyl oder eine Arylgruppe mit bis zu 12 C-Atomen steht,

Y H oder N,

$R^1$ für X = S oder N-$R^3$
Wasserstoff, eine $C_1$-$C_{25}$-Alkyl- oder $C_2$-$C_{25}$-Alkenylgruppe oder ein Phenylrest, der zusätzlich durch eine oder zwei $C_1$-$C_4$-Alkyl-, $C_1$-$C_4$-Alkoxy-, Hydroxyl-, Carboxyl-, Sulfo-, Amino-, $C_1$-$C_4$-Acyl-amino-, Nitro- oder Cyanogruppen oder Chlor- oder Bromatome substituiert sein kann, wobei bei zwei Substituenten diese gleich oder verschieden sein können,

$R^1$ für X = 0
Phenyl, o-, m- oder p-Tolyl, p-Chlorphenyl, m-Nitrophenyl, m-Methoxyphenyl oder m-Methylsulfonylphenyl,

$R^2$ für X = S oder N-$R^3$
Wasserstoff, eine $C_1$-$C_4$-Alkyl-, $C_1$-$C_4$-Alkoxy-, Hydroxyl-, Carboxyl-, Sulfo-, Amino- oder $C_1$-$C_4$-Acylaminogruppe oder ein Chlor- oder Bromatom und

$R^2$ für X = 0
Wasserstoff

als Bleichaktivatoren oder optische Aufheller in Wasch- und Reinigungsmitteln.

2. Verwendung von heterocyclischen Verbindungen I nach Anspruch 1, bei denen X ein Sauerstoffatom bezeichnet, $R^1$ die für den Fall X = O angegebene Bedeutung hat und $R^2$ für Wasserstoff steht.

3. Verwendung von heterocyclischen Verbindungen I nach Anspruch 1, bei denen X Schwefel oder die Gruppierung N-$R^3$ bezeichnet und $R^1$ und $R^2$ die für den Fall X = S oder N-$R^3$ angegebenen Bedeutungen haben.

4. Verwendung von heterocyclischen Verbindungen I nach Anspruch 3, bei denen $R^1$ für Wasserstoff, für $C_1$-$C_{10}$-Alkylgruppe oder für einen Phenylrest steht, der zusätzlich durch eine $C_1$-$C_4$-Alkyl-, $C_1$-$C_4$-Alkoxy-, Hydroxyl-oder Nitrogruppe substituiert sein kann.

5. Verwendung von heterocyclischen Verbindungen I nach Anspruch 3 oder 4, bei denen $R^2$ Wasserstoff, eine $C_1$-$C_4$-Alkyl-, $C_1$-$C_4$-Alkoxy- oder Hydroxylgruppe bedeutet.

6. Verfahren zum Waschen und Reinigen, dadurch gekennzeichnet, daß man dabei heterocyclische Verbindungen der allgemeinen Formel I

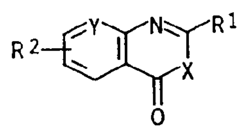

I

in der die Variablen folgende Bedeutung haben

X O, S oder die Gruppierung N-$R^3$, wobei $R^3$ für Wasserstoff, $C_1$-$C_{25}$-Alkyl, $C_1$-$C_{25}$-Acyl oder eine Arylgruppe mit bis zu 12 C-Atomen steht,

Y CH oder N,

$R^1$ für X = S oder N-$R^3$
Wasserstoff, eine $C_1$-$C_{25}$-Alkyl- oder $C_2$-$C_{25}$-Alkenylgruppe oder ein Phenylrest, der zusätzlich durch eine oder zwei $C_1$-$C_4$-Alkyl-, $C_1$-$C_4$-Alkoxy-, Hydroxyl-, Carboxyl-, Sulfo-, Amino-, $C_1$-$C_4$-Acylamino-, Nitro- oder Cyanogruppen oder Chlor- oder Bromatome substituiert sein kann, wobei bei zwei Substituenten diese gleich oder verschieden sein können,

$R^1$ für X = 0
Phenyl, o-, m- oder p-Tolyl, p-Chlorphenyl, m-Nitrophenyl, m-Methoxyphenyl oder m-Methylsulfonylphenyl,

$R^2$ für X = S oder N-$R^3$
Wasserstoff, eine $C_1$-$C_4$-Alkyl-, $C_1$-$C_4$-Alkoxy-, Hydroxyl-, Carboxyl-, Sulfo-, Amino- oder $C_1$-$C_4$-Acylaminogruppe oder ein Chlor- oder Bromatom und

$R^2$ für X = O
Wasserstoff

als Bleichaktivatoren oder optische-Aufheller verwendet.

7. Verfahren zum Waschen und Reinigen nach Anspruch 6, dadurch gekennzeichnet, daß man dabei Verbindungen I verwendet, bei denen X ein Sauerstoffatom bezeichnet, $R^1$ die für den Fall X = O angegebene Bedeutung hat und $R^2$ für Wasserstoff steht.

8. Verfahren zum Waschen und Reinigen nach Anspruch 6, dadurch gekennzeichnet, daß man dabei Verbindungen I verwendet, bei denen X Schwefel oder die Gruppierung N-$R^3$ bezeichnet und $R^1$ und $R^2$ die für den Fall X = S oder N-$R^3$ angegebenen Bedeutungen haben.

9. Verfahren zum Waschen und Reinigen nach Anspruch 8, dadurch gekennzeichnet, daß man dabei Verbindungen I verwendet, bei denen $R^1$ für Wasserstoff, für $C_1$-$C_{10}$-Alkylgruppe oder für einen Phenylrest steht, der zusätzlich durch eine $C_1$-$C_4$-Alkyl-, $C_1$-$C_4$-Alkoxy-, Hydroxyl- oder Nitrogruppe substituiert sein kann.

10. Verfahren zum Waschen und Reinigen nach Anspruch 8 oder 9, dadurch gekennzeichnet, daß man dabei Verbindungen I verwendet, bei denen $R^2$ Wasserstoff, eine $C_1$-$C_4$-Alkyl-, $C_1$-$C_4$-Alkoxy- oder Hydroxylgruppe bedeutet.

11. Verfahren zum Waschen und Reinigen nach Anspruch 8, dadurch gekennzeichnet, daß man dabei Verbindungen I verwendet, bei denen $R^2$ Wasserstoff, eine $C_1$-$C_4$-Alkyl-, $C_1$-$C_4$-Alkoxy- oder Hydroxylgruppe bedeutet.

12. Verfahren zum Waschen und Reinigen nach den Ansprüchen 6 bis 10, dadurch gekennzeichnet, daß man hierzu Zubereitungen verwendet, die 0,1 bis 10 Gew.-%, bezogen auf die Gesamtmenge der Zubereitung, einer oder mehrerer heterocyclischer Verbindungen I enthalten.

13. Wasch- und Reinigungsmittel, dadurch gekennzeichnet, daß sie 0,1 bis 10 Gew.-%, bezogen auf die Gesamtmenge der Zubereitung, einer oder mehrerer heterocyclischer Verbindungen der allgemeinen Formel I gemäß den Ansprüchen 1 bis 5 enthalten.

**Patentansprüche für folgenden Vertragsstaat : ES**

1. Verfahren zum Waschen und Reinigen, dadurch gekennzeichnet, daß man dabei heterocyclische Verbindungen der allgemeinen Formel I

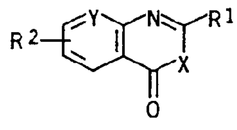

I

in der die Variablen folgende Bedeutung haben

X 0, S oder die Gruppierung N-$R^3$, wobei $R^3$ für Wasserstoff, $C_1$-$C_{25}$-Alkyl, $C_1$-$C_{25}$-Acyl oder eine Arylgruppe mit bis zu 12 C-Atomen steht,

Y CH oder N,

$R^1$ für X = S oder $N-R^3$
Wasserstoff, eine $C_1$-$C_{25}$-Alkyl- oder $C_2$-$C_{25}$-Alkenylgruppe oder ein Phenylrest, der zusätzlich durch eine oder zwei $C_1$-$C_4$-Alkyl-, $C_1$-$C_4$-Alkoxy-, Hydroxyl-, Carboxyl-, Sulfo-, Amino-, $C_1$-$C_4$-Acylamino-, Nitro- oder Cyanogruppen oder Chlor- oder Bromatome substituiert sein kann, wobei bei zwei Substituenten diese gleich oder verschieden sein können,

$R^1$ für X = O
Phenyl, o-, m- oder p-Tolyl, p-Chlorphenyl, m-Nitrophenyl, m-Methoxyphenyl oder m-Methylsulfonylphenyl,

$R^2$ für X = S oder $N-R^3$
Wasserstoff, eine $C_1$-$C_4$-Alkyl-, $C_1$-$C_4$-Alkoxy-, Hydroxyl-, Carboxyl-, Sulfo-, Amino- oder $C_1$-$C_4$-Acylaminogruppe oder ein Chlor- oder Bromatom und

$R^2$ für X = O
Wasserstoff

als Bleichaktivatoren oder optische Aufheller verwendet.

2. Verfahren zum Waschen und Reinigen nach Anspruch 1, dadurch gekennzeichnet, daß man dabei Verbindungen I verwendet, bei denen X ein Sauerstoffatom bezeichnet, $R^1$ die für den Fall X = O angegebene Bedeutung hat und $R^2$ für Wasserstoff steht.

3. Verfahren zum Waschen und Reinigen nach Anspruch 1, dadurch gekennzeichnet, daß man dabei Verbindungen I verwendet, bei denen X Schwefel oder die Gruppierung $N-R^3$ bezeichnet und $R^1$ und $R^2$ die für den Fall X = S oder $N-R^3$ angegebenen Bedeutungen haben.

4. Verfahren zum Waschen und Reinigen nach Anspruch 3, dadurch gekennzeichnet, daß man dabei Verbindungen I verwendet, bei denen $R^1$ für Wasserstoff, für $C_1$-$C_{10}$-Alkylgruppe oder für einen Phenylrest steht, der zusätzlich durch eine $C_1$-$C_4$-Alkyl-, $C_1$-$C_4$-Alkoxy-, Hydroxyl- oder Nitrogruppe substituiert sein kann.

5. Verfahren zum Waschen und Reinigen nach Anspruch 3 oder 4, dadurch gekennzeichnet, daß man dabei Verbindungen I verwendet, bei denen $R^2$ Wasserstoff, eine $C_1$-$C_4$-Alkyl-, $C_1$-$C_4$-Alkoxy- oder Hydroxylgruppe bedeutet.

6. Verfahren zum Waschen und Reinigen nach Anspruch 3, dadurch gekennzeichnet, daß man dabei Verbindungen I verwendet, bei denen $R^2$ Wasserstoff, eine $C_1$-$C_4$-Alkyl-, $C_1$-$C_4$-Alkoxy- oder Hydroxylgruppe bedeutet.

7. Verfahren zum Waschen und Reinigen nach den Ansprüchen 1 bis 6, dadurch gekennzeichnet, daß man hierzu Zubereitungen verwendet, die 0,1 bis 10 Gew.-%, bezogen auf die Gesamtmenge der Zubereitung, einer oder mehrerer heterocyclischer Verbindungen I enthalten.

8. Wasch- und Reinigungsmittel, dadurch gekennzeichnet, daß sie 0,1 bis 10 Gew.-%, bezogen auf die Gesamtmenge der Zubereitung, einer oder mehrerer heterocyclischer Verbindungen der allgemeinen Formel I gemäß Anspruch 1 enthalten.

## Claims

### Claims for the following Contracting States : AT, BE, CH, DE, DK, FR, GB, GR, IT, LI, LU, NL, SE

1. The use of heterocyclic compounds of the general formula I

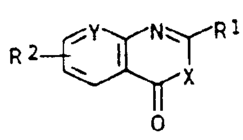

I

where the variables have the following meanings:

X is O, S or the group N-$R^3$, where $R^3$ is hydrogen, $C_1$-$C_{25}$-alkyl, $C_1$-$C_{25}$-acyl or an aryl group of up to 12 carbon atoms,
Y is CH or N,
$R^1$ for X = S or N-$R^3$
is hydrogen, a $C_1$-$C_{25}$-alkyl or $C_2$-$C_{25}$-alkene group or a phenyl radical which may be additionally substituted by one or two $C_1$-$C_4$-alkyl, $C_1$-$C_4$-alkoxy, hydroxyl, carboxyl, sulfo, amino, $C_1$-$C_4$-acylamino, nitro or cyano groups or chlorine or bromine atoms, which substituents, if there are two of them, may be identical or different,
$R^1$ for X = O
is phenyl, o-, m- or p-tolyl, p-chlorophenyl, m-nitrophenyl, m-methoxyphenyl or m-methylsulfonylphenyl,
$R^2$ for X = S or N-$R^3$
is hydrogen, a $C_1$-$C_4$-alkyl, $C_1$-$C_4$-alkoxy, hydroxyl, carboxyl, sulfo, amino or $C_1$-$C_4$-acylamino group or a chlorine or bromine atom, and
$R^2$ for X = O
is hydrogen,
as bleach activators or optical brighteners in washing and cleaning agents.

2. A use of heterocyclic compounds I as claimed in claim 1, wherein X is an oxygen atom, $R^1$ is as defined for the case X = O, and $R^2$ is hydrogen.

3. A use of heterocyclic compounds I as claimed in claim 1, wherein X is sulfur or the group N-$R^3$ and $R^1$ and $R^2$ are each as defined for the case X = S or N-$R^3$.

4. A use of heterocyclic compounds I as claimed in claim 3, wherein $R^1$ is hydrogen, a $C_1$-$C_{10}$-alkyl group or a phenyl radical which may be additionally substituted by $C_1$-$C_4$-alkyl, $C_1$-$C_4$-alkoxy, hydroxyl or nitro.

5. A use of heterocyclic compounds I as claimed in claim 3 or 4, wherein $R^2$ is hydrogen, $C_1$-$C_4$-alkyl, $C_1$-$C_4$-alkoxy or hydroxyl.

6. A process for washing and cleaning, which comprises using heterocyclic compounds of the general formula I

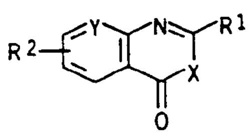

I

where the variables have the following meanings:
X is O, S or the group N-$R^3$, where $R^3$ is hydrogen, $C_1$-$C_{25}$-alkyl, $C_1$-$C_{25}$-acyl or an aryl group of up to 12 carbon atoms,
Y is CH or N,
$R^1$ for X = S or N-$R^3$
is hydrogen, a $C_1$-$C_{25}$-alkyl or $C_2$-$C_{25}$-alkenyl group or a phenyl radical which may be additionally substituted by one or two $C_1$-$C_4$-alkyl, $C_1$-$C_4$-alkoxy, hydroxyl, carboxyl, sulfo, amino, $C_1$-$C_4$-acylamino, nitro or cyano groups or chlorine or bromine atoms, which substituents, if there are two of them, may be identical or different,
$R^1$ for X = O
is phenyl, o-, m- or p-tolyl, p-chlorophenyl, m-nitrophenyl, m-methoxyphenyl or m-methylsulfonylphenyl,
$R^2$ for X = S or N-$R^3$
is hydrogen, a $C_1$-$C_4$-alkyl, $C_1$-$C_4$-alkoxy, hydroxyl, carboxyl, sulfo, amino or $C_1$-$C_4$-acylamino group or a chlorine or bromine atom, and
$R^2$ for X = O
is hydrogen,
as bleach activators or optical brighteners.

7. A process for washing and cleaning as claimed in claim 6, wherein in the compounds I used X is an oxygen

atom, $R^1$ is as defined for the case X = O and $R^2$ is hydrogen.

8. A process for washing and cleaning as claimed in claim 6, wherein in the compounds I used X is sulfur or the group N-$R^3$ and $R^1$ and $R^2$ are each as defined for the case X = S or N-$R^3$.

9. A process for washing and cleaning as claimed in claim 8, wherein in the compounds I used $R^1$ is hydrogen, a $C_1$-$C_{10}$-alkyl group or a phenyl radical which may be additionally substituted by $C_1$-$C_4$-alkyl, $C_1$-$C_4$-alkoxy, hydroxyl or nitro.

10. A process for washing and cleaning as claimed in claim 8 or 9, wherein in the compounds I used $R^2$ is hydrogen, $C_1$-$C_4$-alkyl, $C_1$-$C_4$-alkoxy or hydroxyl.

11. A process for washing and cleaning as claimed in claim 8, wherein in the compounds I used $R^2$ is hydrogen, $C_1$-$C_4$-alkyl, $C_1$-$C_4$-alkoxy or hydroxyl.

12. A process for washing and cleaning as claimed in claim 6 or 7 or 8 or 9 or 10, wherein the formulation used for this purpose contains from 0.1 to 10 % by weight, based on the total amount of the formulation, of one or more heterocyclic compounds I.

13. A washing and cleaning agent containing from 0.1 to 10 % by weight, based on the total amount of the formulation, of one or more heterocyclic compounds of the general formula I as claimed in claim 1 or 2 or 3 or 4 or 5.

**Claims for the following Contracting State : ES**

1. A process for washing and cleaning, which comprises using heterocyclic compounds of the general formula I

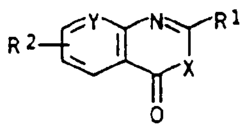

I

where the variables have the following meanings:

X is O, S or the group N-$R^3$, where $R^3$ is hydrogen, $C_1$-$C_{25}$-alkyl, $C_1$-$C_{25}$-acyl or an aryl group of up to 12 carbon atoms,

Y is CH or N,

$R^1$ for X = S or N-$R^3$
is hydrogen, a $C_1$-$C_{25}$-alkyl or $C_2$-$C_{25}$-alkenyl group or a phenyl radical which may be additionally substituted by one or two $C_1$-$C_4$-alkyl, $C_1$-$C_4$-alkoxy, hydroxyl, carboxyl, sulfo, amino, $C_1$-$C_4$-acylamino, nitro or cyano groups or chlorine or bromine atoms, which substituents, if there are two of them, may be identical or different,

$R^1$ for X = O
is phenyl, o-, m- or p-tolyl, p-chlorophenyl, m-nitrophenyl, m-methoxyphenyl or m-methylsulfonylphenyl,

$R^2$ for X = S or N-$R^3$
is hydrogen, a $C_1$-$C_4$-alkyl, $C_1$-$C_4$-alkoxy, hydroxyl, carboxyl, sulfo, amino or $C_1$-$C_4$-acylamino group or a chlorine or bromine atom, and

$R^2$ for X = O
is hydrogen,

as bleach activators or optical brighteners.

2. A process for washing and cleaning as claimed in claim 1, wherein in the compounds I used X is an oxygen atom, $R^1$ is as defined for the case X = O and $R^2$ is hydrogen.

3. A process for washing and cleaning as claimed in claim 1, wherein in the compounds I used X is sulfur or the group N-$R^3$ and $R^1$ and $R^2$ are each as defined for the case X = S or N-$R^3$.

4. A process for washing and cleaning as claimed in claim 3, wherein in the compounds I used $R^1$ is hydrogen, a $C_1$-$C_{10}$-alkyl group or a phenyl radical which may be additionally substituted by $C_1$-$C_4$-alkyl, $C_1$-$C_4$-alkoxy, hydroxyl or nitro.

5. A process for washing and cleaning as claimed in claim 3 or 4, wherein in the compounds I used $R^2$ is hydrogen, $C_1$-$C_4$-alkyl, $C_1$-$C_4$-alkoxy or hydroxyl.

6. A process for washing and cleaning as claimed in claim 3, wherein in the compounds I used $R^2$ is hydrogen, $C_1$-$C_4$-alkyl, $C_1$-$C_4$-alkoxy or hydroxyl.

7. A process for washing and cleaning as claimed in claim 1 or 2 or 3 or 4 or 5 or 6, wherein the formulation used for this purpose contains from 0.1 to 10 % by weight, based on the total amount of the formulation, of one or more heterocyclic compounds I.

8. A washing and cleaning agent containing from 0.1 to 10 % by weight, based on the total amount of the formulation, of one or more heterocyclic compounds of the general formula I as claimed in claim 1.

## Revendications

**Revendications pour les Etats contractants suivants : AT, BE, CH, DE, DK, FR, GB, GR, IT, LI, LU, NL, SE**

1. Utilisation de composés hétérocycliques de formule générale I

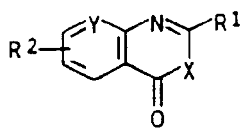

I

dans laquelle les variables ont les significations suivantes:

X représente O, S ou le groupement N-$R^3$, $R^3$ étant mis pour un atome d'hydrogène ou pour un radical alkyle en $C_1$-$C_{25}$, acyle en $C_1$-$C_{25}$ ou aryle renfermant jusqu'à 12 atomes de carbone,

Y représente CH ou N,

$R^1$ représente, dans le cas où X = S ou N-$R^3$,
un atome d'hydrogène, un radical alkyle en $C_1$-$C_{26}$ ou alcényle en $C_2$-$C_{25}$ ou un reste phényle qui peut être substitué en plus par un ou deux groupements alkyle en $C_1$-$C_4$, alcoxy en $C_1$-$C_4$, hydroxy, carboxyle, sulfo, amino, alkylamino en $C_1$-$C_4$, nitro ou cyano ou par un ou deux atomes de chlore ou de brome, ces substituants pouvant être identiques ou différents lorsqu'ils sont au nombre de deux,

$R^1$ représente, dans le cas où X = O,
un reste phényle, o-, m- ou p-tolyle, p-chlorophényle, m-nitrophényle, m-méthoxyphényle ou m-méthylsulfonylphényle,

$R^2$ représente, dans le cas où X = S ou N-$R^3$,
un atome d'hydrogène, un radical alkyle en $C_1$-$C_4$, alcoxy en $C_1$-$C_4$, hydroxy, carboxyle, sulfo, amino ou acylamino en $C_1$-$C_4$ ou un atome de chlore ou de brome, et

$R^2$ représente, dans le cas où X = O,
un atome d'hydrogène,

comme activateurs de blanchiment ou agents d'azurage optique dans des produits de lavage et de nettoyage.

2. Utilisation de composés hétérocycliques I selon la revendication 1, dans lesquels X représente un atome d'oxygène, $R^1$ a la signification indiquée pour le cas où X = O et $R^2$ est mis pour un atome d'hydrogène.

3. Utilisation de composés hétérocycliques I selon la revendication 1, dans lesquels X représente un atome de soufre ou le groupement N-$R^3$, et $R^1$ et $R^2$ ont les significations indiquées pour le cas où X = S ou N-$R^3$.

4. Utilisation de composés hétérocycliques I selon la revendication 3, dans lesquels $R^1$ est mis pour un atome d'hydrogène, pour un radical alkyle en $C_1$-$C_{10}$ ou pour un reste phényle qui peut être substitué en plus par un groupement alkyle en $C_1$-$C_4$, alcoxy en $C_1$-$C_4$, hydroxy ou nitro.

5. Utilisation de composés hétérocycliques I selon la revendication 3 ou 4, dans lesquels $R^2$ représente un atome d'hydrogène ou un radical alkyle en $C_1$-$C_4$, alcoxy en $C_1$-$C_4$ ou hydroxy.

6. Procédé de lavage et de nettoyage, caractérisé en ce qu'on y utilise, comme activateurs de blanchiment ou agents d'azurage optique, des composés hétérocycliques de formule générale I

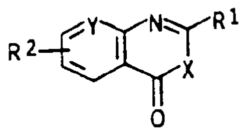

I

dans laquelle les variables ont les significations suivantes:

X représente O, S ou le groupement N-$R^3$, $R^3$ étant mis pour un atome d'hydrogène ou pour un radical alkyle en $C_1$-$C_{25}$, acyle en $C_1$-$C_{25}$ ou aryle renfermant jusqu'à 12 atomes de carbone,

Y représente CH ou N,

$R^1$ représente, dans le cas où X = S ou N-$R^3$,
un atome d'hydrogène, un radical alkyle en $C_1$-$C_{25}$ ou alcényle en $C_2$-$C_{25}$ ou un reste phényle qui peut être substitué en plus par un ou deux groupements alkyle en $C_1$-$C_4$, alcoxy en $C_1$-$C_4$, hydroxy, carboxyle, sulfo, amino, alkylamino en $C_1$-$C_4$, nitro ou cyano ou par un ou deux atomes de chlore ou de brome, ces substituants pouvant être identiques ou différents lorsqu'ils sont au nombre de deux,

$R^1$ représente, dans le cas où X = O,
un reste phényle, o-, m- ou p-tolyle, p-chlorophényle, m-nitrophényle, m-méthoxyphényle ou m-méthylsulfonylphényle,

$R^2$ représente, dans le cas où X = S ou N-$R^3$,
un atome d'hydrogène, un radical alkyle en $C_1$-$C_4$, alcoxy en $C_1$-$C_4$, hydroxy, carboxyle, sulfo, amino ou acylamino en $C_1$-$C_4$ ou un atome de chlore ou de brome, et

$R^2$ représente, dans le cas où X = O,
un atome d'hydrogène.

7. Procédé de lavage et de nettoyage selon la revendication 6, caractérisé en ce qu'on y utilise des composés I dans lesquels X représente un atome d'oxygène, $R^1$ a la signification indiquée pour le cas où X = O et $R^2$ est mis pour un atome d'hydrogène.

8. Procédé de lavage et de nettoyage selon la revendication 6, caractérisé en ce qu'on y utilise des composés I dans lesquels X représente un atome de soufre ou le groupement N-$R^3$, et $R^1$ et $R^2$ ont les significations indiquées pour le cas où X = S ou N-$R^3$.

9. Procédé de lavage et de nettoyage selon la revendication 8, caractérisé en ce qu'on y utilise des composés I dans lesquels $R^1$ est mis pour un atome d'hydrogène, pour un radical alkyle en $C_1$-$C_{10}$ ou pour un reste phényle qui peut être substitué en plus par un groupement alkyle en $C_1$-$C_4$, alcoxy en $C_1$-$C_4$, hydroxy ou nitro.

10. Procédé de lavage et de nettoyage selon la revendication 8 ou 9, caractérisé en ce qu'on y utilise des composés I dans lesquels $R^2$ représente un atome d'hydrogène ou un radical alkyle en $C_1$-$C_4$, alcoxy en $C_1$-$C_4$ ou hydroxy.

11. Procédé de lavage et de nettoyage selon la revendication 8, caractérisé en ce qu'on y utilise des composés I dans lesquels $R^2$ représente un atome d'hydrogène ou un radical alkyle en $C_1$-$C_4$, alcoxy en $C_1$-$C_4$ ou hydroxy.

12. Procédé de lavage et de nettoyage selon l'une quelconque des revendications 6 à 10, caractérisé en ce qu'on utilise à cet effet des préparations qui contiennent de 0,1 à 10% en poids, par rapport à la quantité totale de la préparation, d'un ou de plusieurs composés hétérocycliques I.

13. Produits de lavage et de nettoyage, caractérisés en ce qu'ils contiennent de 0,1 à 10% en poids, par rapport à la quantité totale de la préparation, d'un ou de plusieurs composés hétérocycliques de formule générale I selon l'une quelconque des revendications 1 à 5.

**Revendications pour l'Etat contractant suivant : ES**

1. Procédé de lavage et de nettoyage, caractérisé en ce qu'on y utilise, comme activateurs de blanchiment ou agents d'azurage optique, des composés hétérocycliques de formule générale I

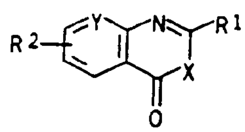

I

dans laquelle les variables ont les significations suivantes:

X représente O, S ou le groupement N-$R^3$, $R^3$ étant mis pour un atome d'hydrogène ou pour un radical alkyle en $C_1$-$C_{25}$, acyle en $C_1$-$C_{25}$ ou aryle renfermant jusqu'à 12 atomes de carbone,

Y représente CH ou N,

$R^1$ représente, dans le cas où X = S ou N-$R^3$,
un atome d'hydrogène, un radical alkyle en $C_1$-$C_{25}$ ou alcényle en $C_2$-$C_{25}$ ou un reste phényle qui peut être substitué en plus par un ou deux groupements alkyle en $C_1$-$C_4$, alcoxy en $C_1$-$C_4$, hydroxy, carboxyle, sulfo, amino, alkylamino en $C_1$-$C_4$, nitro ou cyano ou par un ou deux atomes de chlore ou de brome, ces substituants pouvant être identiques ou différents lorsqu'ils sont au nombre de deux,

$R^1$ représente, dans le cas où X = O,
un reste phényle, o-, m- ou p-tolyle, p-chlorophényle, m-nitrophényle, m-méthoxyphényle ou m-méthylsulfonylphényle,

$R^2$ représente, dans le cas où X = S ou N-$R^3$,
un atome d'hydrogène, un radical alkyle en $C_1$-$C_4$, alcoxy en $C_1$-$C_4$, hydroxy, carboxyle, sulfo, amino ou acylamino en $C_1$-$C_4$ ou un atome de chlore ou de brome, et

$R^2$ représente, dans le cas où X = O,
un atome d'hydrogène.

2. Procédé de lavage et de nettoyage selon la revendication 1, caractérisé en ce qu'on y utilise des composés I dans lesquels X représente un atome d'oxygène, $R^1$ a la signification indiquée pour le cas où X = O et $R^2$ est mis pour un atome d'hydrogène.

3. Procédé de lavage et de nettoyage selon la revendication 1, caractérisé en ce qu'on y utilise des composés I dans lesquels X représente un atome de soufre ou le groupement N-$R^3$, et $R^1$ et $R^2$ ont les significations indiquées pour le cas où X = S ou N-$R^3$.

4. Procédé de lavage et de nettoyage selon la revendication 3, caractérisé en ce qu'on y utilise des composés I dans lesquels $R^1$ est mis pour un atome d'hydrogène, pour un radical alkyle en $C_1$-$C_{10}$ ou pour un reste phényle qui peut être substitué en plus par un groupement alkyle en $C_1$-$C_4$, alcoxy en $C_1$-$C_4$, hydroxy ou nitro.

5. Procédé de lavage et de nettoyage selon la revendication 3 ou 4, caractérisé en ce qu'on y utilise des composés I dans lesquels $R^2$ représente un atome d'hydrogène ou un radical alkyle en $C_1$-$C_4$, alcoxy en $C_1$-$C_4$ ou hydroxy.

6. Procédé de lavage et de nettoyage selon la revendication 3, caractérisé en ce qu'on y utilise des composés I dans lesquels $R^2$ représente un atome d'hydrogène ou un radical alkyle en $C_1$-$C_4$, alcoxy en $C_1$-$C_4$ ou hydroxy.

7. Procédé de lavage et de nettoyage selon l'une quelconque des revendications 1 à 6, caractérisé en ce qu'on utilise à cet effet des préparations qui contiennent de 0,1 à 10% en poids, par rapport à la quantité totale de la préparation, d'un ou de plusieurs composés hétérocycliques I.

8. Produits de lavage et de nettoyage, caractérisés en ce qu'ils contiennent de 0,1 à 10% en poids, par rapport à la quantité totale de la préparation, d'un ou de plusieurs composés hétérocycliques de formule générale I selon la revendication 1.